# EUROPEAN PATENT APPLICATION

(11) **EP 4 368 711 A1**
(43) Date of publication of application: **15.05.2024**
(21) Application number: 21949052.1
(22) Date of filing: 13.10.2021
(51) Int. Cl.: C12N 11/14, C12N 11/10, C12N 11/089, C12N 11/087, C12N 11/082, C12P 19/02, C12R 1/19, C12R 1/125

(54) **PREPARATION METHOD FOR AND APPLICATION OF IMMOBILIZED CELL FOR TAGATOSE PRODUCTION**

(30) Priority: 05.07.2021 CN 202110757673
(71) Applicant: TIANJIN YEAHE BIOTECHNOLOGY CO., LTD, Tianjin, 300308 (CN)
(72) Inventor: MA, Yanhe, Tianjin 300308 (CN); SHI, Ting, Tianjin 300308 (CN); HAN, Pingping, Tianjin 300308 (CN)
(74) Representative: Pharma Patents International AG
(86) International application number: PCT/CN2021/123610
(87) International publication number: WO 2023/279565

(57) **Abstract**

Provided are a preparation method for an immobilized cell for tagatose production and a method for producing tagatose by using the immobilized cell. The preparation method for the immobilized cell comprises: mixing a fermentation broth of Escherichia coli or Bacillus subtilis that expresses α-glucan phosphorylase, phosphoglucomutase, glucose phosphate isomerase, tagatose 6-phosphate epimerase, and tagatose 6-phosphate phosphatase to obtain a fermentation mixture, adding inorganic soil and then performing uniform stirring, then adding a flocculant to flocculate bacteria, subsequently adding a cross-linking agent to cross-link, performing vacuum filtration to obtain a filter cake, using a rotary granulator to extrude the filter cake to granulate into a long strip, then cutting by means of a spherical shot blasting machine into particles having equal lengths, and performing boiling drying to obtain the immobilized cell for tagatose production. According to the present invention, separation and purification steps of an enzyme required in tagatose production are simplified, the recycling rate of the enzyme is improved, and the recycling of the enzyme is achieved.

## Description

### Field of the Invention

The present disclosure relates to the technical field of bioengineering, in particular to the field of tagatose production and preparation.

### Background of the Invention

Tagatose is a naturally occurring rare monosaccharide. It is a keto form of galactose and also an epimer of fructose. It has similar sweetness characteristics with sucrose and provides very fresh and pure sweetness. But it only has one-third of the calories of sucrose, and thus is called a low-calorie sweetener. Researches show that tagatose has important physiologically functional properties such as low calorie, low glycemic index, anti-caries effect, antioxidation, prebiotic actions, improvement of intestinal functions, immune regulation, and use as a drug precursor. It can be widely used in food, beverages, medicine, health care and other fields, having huge economic potential (Oh D-K: Tagatose: properties, applications, and biotechnological processes. App. Microbiol. Biotechnol. 2007, 76:1-8).

Using corn starch, maltodextrin, or sucrose as a raw material at a low cost, Tianjin Institute of Industrial Biotechnology, Chinese Academy of Sciences successfully established a new route for producing tagatose with multiple enzymes *in vitro,* which represented a fundamental change from the existing tagatose production processes (CN106399427A). This new route overcame the disadvantages with the chemical methods for synthesizing tagatose, including high energy consumption, complex products, difficulties in purification, a number of side reactions, and heavy chemical pollution. It also addressed the defects with the existing biological methods for producing tagatose, including the expensive raw material which limited the use thereof, a low conversion rate and a complicated separation process. Further, Tianjin Institute of Industrial Biotechnology, Chinese Academy of Sciences adopted a method for preparing tagatose with a whole cell for catalysis, using a low-cost raw material such as corn starch, cellulose, maltodextrin or sucrose (CN107988286A). This method saved the steps for purifying multiple enzymes, cut the production cost to a low level, reduced the environmental pollution and improved the tagatose yield. To ensure the safety of food preparations in production, the Tianjin Institute of Industrial Biotechnology made further improvements and provided a method for producing tagatose at a high concentration by catalyzing high-concentration starch with a whole cell of *Bacillus subtilis* (CN112342179B). This method immobilized the permeabilized *Bacillus subtilis* cell to obtain an immobilized whole cell, and then used the cell to produce tagatose. It achieved recycling of the whole cells and thus reduced the production cost. However, the analysis from researches showed that this method still had some shortcomings in preparing an immobilized cell. Firstly, this method required a plurality of steps such as cell collection, resuspension, and permeabilization before immobilization. Secondly, this method used a permeabilized cell for granulation and immobilization, wherein the permeabilization of the cell could easily cause leakage of a heterologous protein expressed in the cell, which led to the loss of the heterologous protein and reduction of enzyme immobilization efficiency during the immobilization process. Thirdly, the method obtained the immobilized enzyme particle by simply extruding to achieve granulation, and thus the obtained particle was not of a uniform size.

Therefore, there is an urgent need to develop a method to obtain immobilized cells of a uniform size in a simple and convenient way. This method should simplify the process of providing enzymes, and avoid or reduce the enzyme leakage or loss during immobilization of a permeable cell to improve the enzyme immobilization efficiency. It should also achieve enzyme recycling to further reduce the tagatose production cost and enable the tagatose production on an industrial scale.

### Summary of the Invention

The existing methods for producing tagatose with immobilized cells have many problems, for example, the need of a plurality of steps for producing enzymes, enzyme loss and reduced immobilization efficiency due to permeabilization, and an uneven particle size of prepared immobilized enzymes. In view of these problems, an object of the present disclosure is to provide a method for producing tagatose with immobilized cells. It simplifies the steps for providing enzymes and facilitates the separation and purification of the product from the enzymes during tagatose production. It achieves recycling of multiple enzymes, reduces the tagatose production cost and enables the tagatose production on an industrial scale.

In order to solve the above technical problems, the present disclosure adopts the following technical solution:
A method for preparing an immobilized cell for tagatose production, characterized in that the method comprises the steps of:
fermenting to obtain fermentation broths of *Escherichia coli* or *Bacillus subtilis* expressing α-glucan phosphorylase, phosphoglucomutase, phosphoglucose isomerase, tagatose 6-phosphate epimerase, or tagatose 6-phosphate phosphatase respectively, and mixing the above fermentation broths to obtain a fermentation mixture;
adding an inorganic soil to the fermentation mixture, and stirring homogeneously;
further adding a flocculant to the fermentation mixture to flocculate bacteria, and then adding a cross-linking agent for cross-linking;
filtering under vacuum to obtain a filter cake, extruding the filter cake to granulate into a strip with a rotary granulator, and then breaking into particles having a uniform length with a spherical shot blasting machine; and
subjecting the resulting particles to boiling drying to obtain the immobilized cell for tagatose production.

In an embodiment, the present disclosure adopts the following technical solution:
A method for preparing immobilized cells for tagatose production, characterized in that the method comprises the steps of:
fermenting to obtain fermentation broths of *Escherichia coli* or *Bacillus subtilis* expressing α-glucan phosphorylase, phosphoglucomutase, phosphoglucose isomerase, tagatose 6-phosphate epimerase, or tagatose 6-phosphate phosphatase respectively, and mixing the above fermentation broths to obtain a fermentation mixture;
adding 1-10% w/v of inorganic soil to the fermentation mixture, and stirring homogeneously;
further adding 0.1-2% w/v of flocculant to the fermentation mixture to flocculate bacteria, then adding 0.05-3% v/v of cross-linking agent, and cross-linking for 1-4 hours;
filtering under vacuum to obtain a filter cake, extruding the filter cake to granulate into a strip with a rotary granulator, and then breaking the strip of immobilized cells into particles having a uniform length with a spherical shot blasting machine; and
subjecting the resulting particles to boiling drying to obtain the immobilized cells for tagatose production, wherein the temperature at an air inlet for the boiling drying is controlled at 60-90°C.

The fermentation broths are prepared by a method known in the art. The fermenting can be carried out using any culture medium for exogenous protein expression, including but not limited to LB medium, SR medium, or TB medium.

Preferably, the α-glucan phosphorylase, the phosphoglucomutase, the phosphoglucose isomerase, the tagatose 6-phosphate epimerase, or the tagatose 6-phosphate phosphatase is thermostable α-glucan phosphorylase, thermostable phosphoglucomutase, thermostable phosphoglucose isomerase, thermostable tagatose 6-phosphate epimerase, or thermostable tagatose 6-phosphate phosphatase respectively.

Specifically, the thermostable α-glucan phosphorylase refers to an enzyme having the function of phosphorylating starch into glucose-1-phosphate (G1P) at 40°C or above, 45°C or above, 50°C or above, 55°C or above, 60°C or above, 65°C or above, 70°C or above, 75°C or above, or 80°C or above. Further preferably, the thermostable α-glucan phosphorylase is derived from a thermophilic microorganism, for example, *Geobacillus kaustophilus, Geobacillus stearothermophilus, Thermotoga maritima, Pseudothermotoga thermarum, Thermococcus kodakarensis, Archaeoglobus fulgidus, Thermoanaerobacter indiensis, Dictyoglomus thermophilum, Caldicellulosiruptor kronotskyensis, Clostridium thermocellum, Caldilinea aerophila, Pyrococcus furiosus, Thermus thermophilus, Methanothermobacter marburgensis,* or *Archaeoglobus profundus;* or the thermostable α-glucan phosphorylase has an amino acid sequence which is at least 70%, preferably at least 80%, more preferably at least 90%, or most preferably at least 95% identical to that of the thermostable α-glucan phosphorylase derived from the thermophilic microorganism. More preferably, the thermostable α-glucan phosphorylase is derived from *Thermococcus kodakarensis.*

Specifically, the thermostable phosphoglucomutase refers to an enzyme having the function of translocating glucose-1-phosphate (G1P) into glucose-6-phosphate (G6P) at 40°C or above, 45°C or above, 50°C or above, 55°C or above, 60°C or above, 65°C or above, 70°C or above, 75°C or above, or 80°C or above. Further preferably, the thermostable phosphoglucomutase is derived from a thermophilic microorganism, for example, *Geobacillus kaustophilus, Geobacillus stearothermophilus, Thermotoga maritima, Pseudothermotoga thermarum, Thermococcus kodakarensis, Archaeoglobus fulgidus, Thermoanaerobacter indiensis, Dictyoglomus thermophilum, Caldicellulosiruptor kronotskyensis, Clostridium thermocellum, Caldilinea aerophila, Pyrococcus furiosus, Thermus thermophilus, Methanothermobacter marburgensis,* or *Archaeoglobus profundus;* or the thermostable phosphoglucomutase has an amino acid sequence which is at least 70%, preferably at least 80%, more preferably at least 90%, or most preferably at least 95% identical to that of the thermostable phosphoglucomutase derived from the thermophilic microorganism. More preferably, the thermostable phosphoglucomutase is derived from *Thermococcus kodakarensis.*

Specifically, the thermostable phosphoglucose isomerase refers to an enzyme having the function of translocating glucose-6-phosphate (G6P) into fructose-6-phosphate (F6P) at 40°C or above, 45°C or above, 50°C or above, 55°C or above, 60°C or above, 65°C or above, 70°C or above, 75°C or above, or 80°C or above. Further preferably, the thermostable phosphoglucose isomerase is derived from a thermophilic microorganism, for example, *Geobacillus kaustophilus, Geobacillus stearothermophilus, Thermotoga maritima, Pseudothermotoga thermarum, Thermococcus kodakarensis, Archaeoglobus fulgidus, Thermoanaerobacter indiensis, Dictyoglomus thermophilum, Caldicellulosiruptor kronotskyensis, Clostridium thermocellum, Caldilinea aerophila, Pyrococcus furiosus, Thermus thermophilus, Methanothermobacter marburgensis,* or *Archaeoglobus profundus;* or the thermostable phosphoglucose isomerase has an amino acid sequence which is at least 70%, preferably at least 80%, more preferably at least 90%, or most preferably at least 95% identical to that of the thermostable phosphoglucose isomerase derived from the thermophilic microorganism. More preferably, the thermostable phosphoglucose isomerase is derived from *Thermus thermophilus.*

Specifically, the thermostable tagatose 6-phosphate epimerase refers to an enzyme having the function of isomerizing fructose-6-phosphate (F6P) into tagatose-6-phosphate (T6P) at 40°C or above, 45°C or above, 50°C or above, 55°C or above, 60°C or above, 65°C or above, 70°C or above, 75°C or above, or 80°C or above. Further preferably, the thermostable tagatose 6-phosphate epimerase is derived from a thermophilic microorganism, for example, *Geobacillus kaustophilus, Geobacillus stearothermophilus, Thermotoga maritima, Pseudothermotoga thermarum, Thermococcus kodakarensis, Archaeoglobus fulgidus, Thermoanaerobacter indiensis, Dictyoglomus thermophilum, Caldicellulosiruptor kronotskyensis, Clostridium thermocellum, Caldilinea aerophila, Pyrococcus furiosus, Thermus thermophilus, Methanothermobacter marburgensis,* or *Archaeoglobus profundus;* or the thermostable tagatose 6-phosphate epimerase has an amino acid sequence which is at least 70%, preferably at least 80%, more preferably at least 90%, or most preferably at least 95% identical to that of the thermostable tagatose 6-phosphate epimerase derived from the thermophilic microorganism. More preferably, the thermostable tagatose 6-phosphate epimerase is derived from *Thermoanaerobacter indiensis.*

Specifically, the tagatose 6-phosphate phosphatase refers to an enzyme having the function of removing a phosphate group of tagatose-6-phosphate (T6P) to produce tagatose at 40°C or above, 45°C or above, 50°C or above, 55°C or above, 60°C or above, 65°C or above, 70°C or above, 75°C or above, or 80°C or above. Further preferably, the tagatose 6-phosphate phosphatase is derived from a thermophilic microorganism, for example, *Geobacillus kaustophilus, Geobacillus stearothermophilus, Thermotoga maritima, Pseudothermotoga thermarum, Thermococcus kodakarensis, Archaeoglobus fulgidus, Thermoanaerobacter indiensis, Dictyoglomus thermophilum, Caldicellulosiruptor kronotskyensis, Clostridium thermocellum, Caldilinea aerophila, Pyrococcus furiosus, Thermus thermophilus, Methanothermobacter marburgensis,* or *Archaeoglobus profundus*; or the tagatose 6-phosphate phosphatase has an amino acid sequence which is at least 70%, preferably at least 80%, more preferably at least 90%, or most preferably at least 95% identical to that of the tagatose 6-phosphate phosphatase derived from the thermophilic microorganism. More preferably, the tagatose 6-phosphate phosphatase is derived from *Archaeoglobus fulgidus.*

Further preferably, the wet bacteria expressing the thermostable α-glucan phosphorylase, the thermostable phosphoglucomutase, the thermostable phosphoglucose isomerase, the thermostable tagatose 6-phosphate epimerase, and the thermostable tagatose 6-phosphate phosphatase respectively are mixed in a ratio of (0.1-10) : (0.1-10) : (0.1-10) : (0.1-10) : (0.1-10), and the bacteria suspension obtained by mixinghas an OD600 of 10-150.

Further, the inorganic soil includes but is not limited to montmorillonite, diatomite, kaolin or bentonite; preferably, the inorganic soil is diatomite.

Further, the flocculant includes but is not limited to polyethyleneimine, chitosan, poly(diallyldimethylammonium chloride) (PDADMAC), or polyacrylamide; preferably, the flocculant is polyethyleneimine or PDADMAC; preferably, the polyethyleneimine has a molecular weight of 600-70,000.

Further, the cross-linking agent includes but is not limited to glutaraldehyde, tris(hydroxymethyl)phosphine, N,N-methylenebisacrylamide, or epichlorohydrin; preferably, the cross-linking agent is glutaraldehyde.

The method further comprises a step of sieving the obtained immobilized cells to obtain morphologically uniform immobilized cells.

Accordingly, the present disclosure also provides a method for producing tagatose with an immobilized cell, characterized in that the method comprises converting starch or a starch derivative into tagatose with the above immobilized cell.

Further, after the reaction is completed, a step of filtering and recovering the immobilized cell is also included.

In an embodiment, the reaction system for biological conversion comprises 50-300 g/L of starch or starch derivative, a buffer at pH 5.0-8.0, 10-50 mM inorganic phosphate, 3-7 mM divalent magnesium ions, and the immobilized cell.

Further, the buffer can be an HEPES buffer, a phosphate buffer, a Tris buffer, or an acetate buffer, or the like. The inorganic phosphate can be sodium phosphate or potassium phosphate.

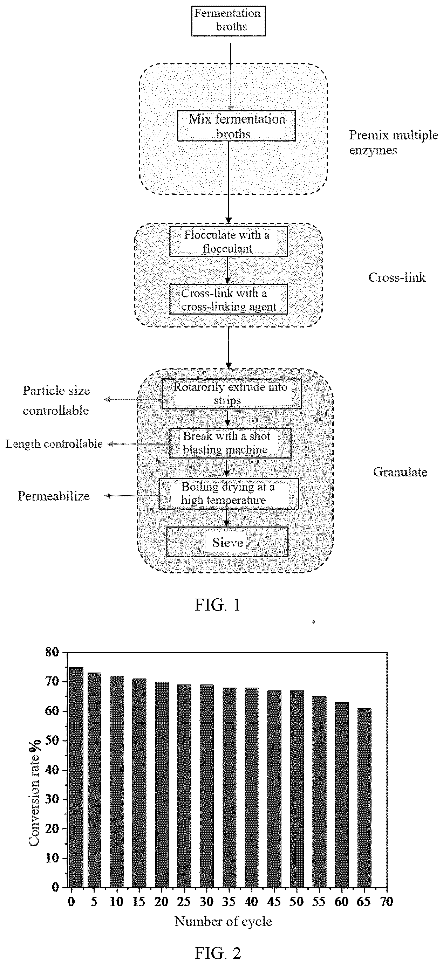

Compared with the prior art, the present disclosure has the following beneficial effects. Compared with the use of multi-enzymes to catalyze reactions in the prior art, the present method for producing tagatose with immobilized cells simplifies the enzyme providing or preparing process, addresses the difficulties in separating multi-enzymes from the product, and facilitates the separation and purification of the product tagatose. The immobilized cells can be separated from the reaction solution simply by filtration. Compared with the use of whole cells to catalyze reactions in the prior art, the present disclosure further simplifies the separation of enzymes from the product, enables the repeated use of enzymes, improves the recycling rate of cells and reduces the tagatose production cost. In addition, the recycling of cells reduces the environmental pollution by cutting multiple times of fermentation, and simplifies the operation steps. Importantly, in the present disclosure, after the fermentation broths comprising expressed enzymes are prepared and mixed, the fermentation mixture is directly used for later steps including granulation, saving the steps of collecting bacteria from the fermentation broths, and resuspending and permeabilizing the bacteria (since the step of recovering the bacteria from the fermentation broths is omitted, the immobilization process is simplified and the operability of the process is improved; the step of permeabilizing cells is also omitted). As the fermentation mixture is directly used for immobilization, the membranes and the walls of the cells suffer from little damage, and the expressed enzymes are seldom leaked after immobilization. Thus, a higher enzyme immobilization efficiency can be obtained. In the present disclosure, after the immobilization which results in immobilized cells, a rotary extruding granulator is used to prepare strips with a controllable cross-sectional size, and then a spherical shot blasting machine is used to break the prepared strips into particles having a uniform length. Subsequently, the particles are subjected to boiling drying at a high temperature (to achieve cell permeabilization), and sieved to obtain immobilized enzyme particles having a uniform size, which can be more effective in tagatose production. The granulation process adopted in the present disclosure not only facilitates the subsequent permeabilization and helps ensure the uniformity of the granules, but also makes the previous steps simpler for bacteria collection (see the specific process of preparing immobilized cells according to the present disclosure as shown in Figure 1). Experiments showed that the present disclosure achieved a significant effect. When the immobilized *Bacillus subtilis* of the present disclosure were used to catalyze consecutive rounds of reactions, the initial product yield could reach up to 75%, and even they were used to carry out catalysis for 65 consecutive batches, the product yield could still be maintained at 61%. When the immobilized *Escherichia coli* of the present disclosure were used to catalyze consecutive rounds of reactions, the initial product yield could reach up to 74%, and even they were used to carry out catalysis for 65 consecutive batches, the product yield could still be maintained at 60%.

### Brief Description of the Drawings

Figure 1 shows a schematic diagram of a specific process of preparing immobilized cells according to the present disclosure.
Figure 2 shows the effect of the immobilized *Bacillus subtilis* in producing tagatose in Example 3.
Figure 3 shows the effect of the immobilized *Escherichia coli* in producing tagatose in Example 11.
Figure 4 shows the effect of the *Bacillus subtilis* in producing tagatose in Comparative Example 1.
Figure 5 shows the effect of the *Escherichia coli* in producing tagatose in Comparative Example 2.

### Detailed Description of the Invention

### Definition of Terms

When used in conjunction with the term "comprising", "comprise(s)", or "comprised" in the claims and/or description, the term "a" or "an" may refer to "one", and may also refer to "one or more", "at least one", or "one or more than one".

As used in the claims and description, the term "comprising", "having", "including" or "containing", or any grammatical variation thereof is inclusive or open-ended, and does not exclude any additional element or step which is not recited.

Although the term "or" is supported by the present disclosure to refer to only an alternative or refer to the meaning of "and/or", the term "or" in the claims refers to "and/or", unless it is expressly indicated as referring to only an alternative or the alternatives are mutually exclusive.

Unless otherwise defined, all technical or scientific terms used herein have the same meaning as commonly understood by one of ordinary skill in the art to which this disclosure belongs. Although any method or material similar or equivalent to that described herein can be used in the practice or testing of the present disclosure, the methods and materials described herein are preferred.

In order to further illustrate the technical solutions adopted by the present disclosure and the effects thereof, the technical solutions of the present disclosure are further described through specific Examples below. However, it should be understood that the described Examples are exemplary only and do not pose any limitation to the scope of the present disclosure. Unless otherwise specified, the experimental techniques and experimental methods used in the present examples are conventional techniques and methods. For example, the conditions which are not specifically indicated in the experimental methods in the following Examples are usually conventional conditions such as those described in Sambrook et al., Molecular Cloning: Laboratory Handbook, New York: Cold Spring Harbor Laboratory Press, 1989, or conditions as recommended by the manufacturer(s). Unless otherwise specified, the materials, reagents and other substances used in the Examples are available commercially. It is understood by one skilled in the art that the details and forms of the technical solutions of the present disclosure can be modified or substituted without departing from the spirit and scope of the present disclosure, and these modifications or substitutions fall within the protection scope of the present disclosure.

### Example 1: Preparation of whole cells of Bacillus subtilis

Recombinant *Bacillus subtilis* strains (SCK6 was selected as the starting strain, see CN112342179B) expressing a gene of thermostable α-glucan phosphorylase, a gene of thermostable phosphoglucomutase, a gene of thermostable phosphoglucose isomerase, a gene of thermostable tagatose 6-phosphate epimerase, or a gene of thermostable tagatose 6-phosphate phosphatase were selected respectively, inoculated into LB medium respectively, and cultured overnight at 37°C with shaking. Then the culture was transferred to LB medium at an inoculum volume of 1%, and cultured overnight at 37°C with shaking to obtain a fermentation broth of *Bacillus subtilis* expressing thermostable α-glucan phosphorylase, a fermentation broth of *Bacillus subtilis* expressing thermostable phosphoglucomutase, a fermentation broth of *Bacillus subtilis* expressing thermostable phosphoglucose isomerase, a fermentation broth of *Bacillus subtilis* expressing thermostable tagatose 6-phosphate epimerase, and a fermentation broth of *Bacillus subtilis* expressing thermostable tagatose 6-phosphate phosphatase, respectively.

### Example 2: Production of tagatose with immobilized Bacillus subtilis

The fermentation broth of *Bacillus subtilis* expressing thermostable α-glucan phosphorylase, the fermentation broth of *Bacillus subtilis* expressing thermostable phosphoglucomutase, the fermentation broth of *Bacillus subtilis* expressing thermostable phosphoglucose isomerase, the fermentation broth of *Bacillus subtilis* expressing thermostable tagatose 6-phosphate epimerase, and the fermentation broth of *Bacillus subtilis* expressing thermostable tagatose 6-phosphate phosphatase prepared in Example 1 were mixed in a ratio of 1 : 1 : 1 : 1 : 1 according to OD600, so that the OD600 = 100. And 1% w/v of montmorillonite was added to the bacteria suspension, and stirred homogeneously. Subsequently, 0.5% w/v of the aqueous solution of polyethyleneimine having a molecular weight of 10,000 was added and flocculation was carried out at room temperature. Then, 2% v/v of glutaraldehyde aqueous solution was added and cross-linking was carried out at room temperature for 2 hours. Filtration was carried out under vacuum to obtain a filter cake. The filter cake was extruded and thus granulated into strips having a particle size of 1.0 mm with a rotary granulator. Then the strips were broken into particles having a uniform length with a spherical shot blasting machine. The resulting immobilized cell particles were subjected to boiling drying at 60°C to obtain immobilized cells.

In a reaction system having a volume of 1 L, starch at a final concentration of 100 g/L, a 50 mM sodium phosphate buffer (pH 7.0) and the immobilized *Bacillus subtilis* was respectively added to achieve OD600 = 20. Reaction was allowed to carried out in a water bath in a shaker at 70°C. During reaction, the content of tagatose was analyzed by high performance liquid chromatography (HPLC). After the reaction was completed, the immobilized *Bacillus subtilis* was collected by simple filtration and washed with buffer solution before proceeding to the next batch of reaction. Experimental results showed that when the immobilized *Bacillus subtilis* was used to catalyze consecutive rounds of reactions, the initial product yield reached up to 75%, and as the catalysis was carried out for 65 consecutive batches, the product yield was still maintained at 58%.

### Example 3: Production of tagatose with immobilized Bacillus subtilis cells

The fermentation broth of *Bacillus subtilis* expressing thermostable α-glucan phosphorylase, the fermentation broth of *Bacillus subtilis* expressing thermostable phosphoglucomutase, the fermentation broth of *Bacillus subtilis* expressing thermostable phosphoglucose isomerase, the fermentation broth of *Bacillus subtilis* expressing thermostable tagatose 6-phosphate epimerase, and the fermentation broth of *Bacillus subtilis* expressing thermostable tagatose 6-phosphate phosphatase prepared in Example 1 were mixed in a ratio of 1 : 1 : 1 : 1 : 1 according to OD600, so that the OD600 = 100. And 5% w/v of diatomite was added to the bacteria suspension, and stirred homogeneously. Subsequently, 0.1% w/v of the aqueous solution of polyethyleneimine having a molecular weight of 70,000 was added and flocculation was carried out at room temperature. Then, 1% v/v of glutaraldehyde aqueous solution was added and cross-linking was carried out at room temperature for 2 hours. Filtration was carried out under vacuum to obtain a filter cake. The filter cake was extruded and thus granulated into strips having a particle size of 3.0 mm with a rotary granulator. Then the strips were broken into particles having a uniform length with a spherical shot blasting machine. The resulting immobilized cell particles were subjected to boiling drying at 75°C to obtain immobilized cells.

Tagatose was produced using the method of Example 2. Experimental results showed that when the immobilized *Bacillus subtilis* was used to catalyze consecutive rounds of reactions, the initial product yield reached up to 75%, and as the catalysis was carried out for 65 consecutive batches, the product yield was still maintained at 61%.

### Example 4: Production of tagatose with immobilized Bacillus subtilis

The fermentation broth of *Bacillus subtilis* expressing thermostable α-glucan phosphorylase, the fermentation broth of *Bacillus subtilis* expressing thermostable phosphoglucomutase, the fermentation broth of *Bacillus subtilis* expressing thermostable phosphoglucose isomerase, the fermentation broth of *Bacillus subtilis* expressing thermostable tagatose 6-phosphate epimerase, and the fermentation broth of *Bacillus subtilis* expressing thermostable tagatose 6-phosphate phosphatase prepared in Example 1 were mixed in a ratio of 1 : 1 : 1 : 1 : 1 according to OD600, so that the OD600 was at about 100. And 10% w/v of diatomite was added to the bacteria suspension, and stirred homogeneously. Subsequently, 1% w/v of poly(diallyldimethylammonium chloride) (PDADMAC) aqueous solution was added and flocculation was carried out at room temperature. Then, 0.05% v/v of glutaraldehyde aqueous solution was added and cross-linking was carried out at room temperature for 3 hours. Filtration was carried out under vacuum to obtain a filter cake. The filter cake was extruded and thus granulated into strips having a particle size of 1.0 mm with a rotary granulator. Then the strips were broken into particles having a uniform length with a spherical shot blasting machine. The resulting immobilized cell particles were subjected to boiling drying at 90°C to obtain immobilized cells.

Tagatose was produced using the method of Example 2. Experimental results as shown in Figure 1 demonstrated that when the immobilized *Bacillus subtilis* was used to catalyze consecutive rounds of reactions, the initial product yield reached up to 75%, and as the catalysis was carried out for 65 consecutive batches, the product yield was still maintained at 59%.

### Example 5: Production of tagatose with immobilized Bacillus subtilis cells

The fermentation broth of *Bacillus subtilis* expressing thermostable α-glucan phosphorylase, the fermentation broth of *Bacillus subtilis* expressing thermostable phosphoglucomutase, the fermentation broth of *Bacillus subtilis* expressing thermostable phosphoglucose isomerase, the fermentation broth of *Bacillus subtilis* expressing thermostable tagatose 6-phosphate epimerase, and the fermentation broth of *Bacillus subtilis* expressing thermostable tagatose 6-phosphate phosphatase prepared in Example 1 were mixed in a ratio of 1 : 1 : 1 : 5 : 5 according to OD600, so that the OD600 was at about 100. And 5% w/v of diatomite was added to the bacteria suspension, and stirred homogeneously. Subsequently, 0.5% w/v of polyethyleneimine having a molecular weight of 600 was added and flocculation was carried out at room temperature. Then, 0.3% v/v of glutaraldehyde aqueous solution was added and cross-linking was carried out at room temperature for 3 hours. Filtration was carried out under vacuum to obtain a filter cake. The filter cake was extruded and thus granulated into strips having a particle size of 0.4 mm with a rotary granulator. Then the strips were broken into particles having a uniform length with a spherical shot blasting machine. The resulting immobilized cell particles were subjected to boiling drying at 80°C to obtain immobilized cells.

Tagatose was produced using the method of Example 2. Experimental results showed that when the immobilized *Bacillus subtilis* was used to catalyze consecutive rounds of reactions, the initial product yield reached up to 75%, and as the catalysis was carried out for 65 consecutive batches, the product yield was maintained at 56%.

### Example 6: Production of tagatose with immobilized Bacillus subtilis cells

The fermentation broth of *Bacillus subtilis* expressing thermostable α-glucan phosphorylase, the fermentation broth of *Bacillus subtilis* expressing thermostable phosphoglucomutase, the fermentation broth of *Bacillus subtilis* expressing thermostable phosphoglucose isomerase, the fermentation broth of *Bacillus subtilis* expressing thermostable tagatose 6-phosphate epimerase, and the fermentation broth of *Bacillus subtilis* expressing thermostable tagatose 6-phosphate phosphatase prepared in Example 1 were mixed in a ratio of 1 : 1 : 1 : 1 : 1 according to OD600, so that the OD600 was at about 100. And 6% w/v of diatomite was added to the bacteria suspension, and stirred homogeneously. Subsequently, 1% w/v of polyethyleneimine having a molecular weight of 600 was added and flocculation was carried out at room temperature. Then, 1% v/v of tris(hydroxymethyl)phosphine aqueous solution was added and cross-linking was carried out at room temperature for 3 hours. Filtration was carried out under vacuum to obtain a filter cake. The filter cake was extruded and thus granulated into strips having a particle size of 0.4 mm with a rotary granulator. Then the strips were broken into particles having a uniform length with a spherical shot blasting machine. The resulting immobilized cell particles were subjected to boiling drying at 80°C to obtain immobilized cells.

Tagatose was produced using the method of Example 2. Experimental results showed that when the immobilized *Bacillus subtilis* was used to catalyze consecutive rounds of reactions, the initial product yield reached up to 75%, and as the catalysis was carried out for 65 consecutive batches, the product yield was maintained at 59%.

### Example 7: Production of tagatose with immobilized Bacillus subtilis cells

The fermentation broth of *Bacillus subtilis* expressing thermostable α-glucan phosphorylase, the fermentation broth of *Bacillus subtilis* expressing thermostable phosphoglucomutase, the fermentation broth of *Bacillus subtilis* expressing thermostable phosphoglucose isomerase, the fermentation broth of *Bacillus subtilis* expressing thermostable tagatose 6-phosphate epimerase, and the fermentation broth of *Bacillus subtilis* expressing thermostable tagatose 6-phosphate phosphatase prepared in Example 1 were mixed in a ratio of 1 : 1 : 1 : 1 : 1 according to OD600, so that the OD600 = 100. And 3% w/v of diatomite was added to the bacteria suspension, and stirred homogeneously. Subsequently, 0.5% w/v of polyacrylamide was added and flocculation was carried out at room temperature. Then, 2.0% v/v of N,N-methylenebisacrylamide aqueous solution was added and cross-linking was carried out at room temperature for 3 hours. Filtration was carried out under vacuum to obtain a filter cake. The filter cake was extruded and thus granulated into strips having a particle size of 1.0 mm with a rotary granulator. Then the strips were broken into particles having a uniform length with a spherical shot blasting machine. The resulting immobilized cell particles were subjected to boiling drying at 90°C to obtain immobilized cells.

Tagatose was produced using the method of Example 2. Experimental results showed that when the immobilized *Bacillus subtilis* was used to catalyze consecutive rounds of reactions, the initial product yield reached up to 75%, and as the catalysis was carried out for 65 consecutive batches, the product yield was maintained at 57%.

### Example 8: Production of tagatose with immobilized Bacillus subtilis cells

The fermentation broth of *Bacillus subtilis* expressing thermostable α-glucan phosphorylase, the fermentation broth of *Bacillus subtilis* expressing thermostable phosphoglucomutase, the fermentation broth of *Bacillus subtilis* expressing thermostable phosphoglucose isomerase, the fermentation broth of *Bacillus subtilis* expressing thermostable tagatose 6-phosphate epimerase, and the fermentation broth of *Bacillus subtilis* expressing thermostable tagatose 6-phosphate phosphatase prepared in Example 1 were mixed in a ratio of 1 : 1 : 1 : 1 : 1 according to OD600, so that the OD600 = 100. And 1% w/v of diatomite was added to the bacteria suspension, and stirred homogeneously. Subsequently, 0.1% w/v of the aqueous solution of polyethyleneimine having a molecular weight of 70,000 was added and flocculation was carried out at room temperature. Then, 0.5% v/v of epichlorohydrin aqueous solution was added and cross-linking was carried out at room temperature for 3 hours. Filtration was carried out under vacuum to obtain a filter cake. The filter cake was extruded and thus granulated into strips having a particle size of 1.0 mm with a rotary granulator. Then the strips were broken into particles having a uniform length with a spherical shot blasting machine. The resulting immobilized cell particles were subjected to boiling drying at 75°C to obtain immobilized cells.

Tagatose was produced using the method of Example 2. Experimental results showed that when the immobilized *Bacillus subtilis* was used to catalyze consecutive rounds of reactions, the initial product yield reached up to 75%, and as the catalysis was carried out for 65 consecutive batches, the product yield was maintained at 58%.

### Example 9: Preparation of whole cells of Escherichia coli

Recombinantly engineered *Escherichia coli* strains (BL21(DE3) was selected as the starting strain, see CN107988286B) expressing a gene of thermostable α-glucan phosphorylase, a gene of thermostable phosphoglucomutase, a gene of thermostable phosphoglucose isomerase, a gene of thermostable tagatose 6-phosphate epimerase, or a gene of thermostable tagatose 6-phosphate phosphatase were selected respectively, inoculated into LB medium respectively, and cultured overnight at 37°C with shaking. Then the culture was transferred to LB medium at an inoculum volume of 1%, added with IPTG for induction at 18°C and cultured overnight with shaking to obtain a fermentation broth of *Escherichia coli* expressing thermostable α-glucan phosphorylase, a fermentation broth of *Escherichia coli* expressing thermostable phosphoglucomutase, a fermentation broth of *Escherichia coli* expressing thermostable phosphoglucose isomerase, a fermentation broth of *Escherichia coli* expressing thermostable tagatose 6-phosphate epimerase, and a fermentation broth of *Escherichia coli* expressing thermostable tagatose 6-phosphate phosphatase, respectively.

### Example 10: Production of tagatose with immobilized Escherichia coli cells

The fermentation broth of *Escherichia coli* expressing thermostable α-glucan phosphorylase, the fermentation broth of *Escherichia coli* expressing thermostable phosphoglucomutase, the fermentation broth of *Escherichia coli* expressing thermostable phosphoglucose isomerase, the fermentation broth of *Escherichia coli* expressing thermostable tagatose 6-phosphate epimerase, and the fermentation broth of *Escherichia coli* expressing thermostable tagatose 6-phosphate phosphatase prepared in Example 9 were mixed in a ratio of 1 : 1 : 1 : 1 : 1 according to OD600, so that the OD600 was at about 100. And 1% w/v of montmorillonite was added to the bacteria suspension, and stirred homogeneously. Subsequently, 0.5% w/v of the aqueous solution of polyethyleneimine having a molecular weight of 10,000 was added and flocculation was carried out at room temperature. Then, 2% v/v of glutaraldehyde aqueous solution was added and cross-linking was carried out at room temperature for 2 hours. Filtration was carried out under vacuum to obtain a filter cake. The filter cake was extruded and thus granulated into strips having a particle size of 1.0 mm with a rotary granulator. Then the strips were broken into particles having a uniform length with a spherical shot blasting machine. The resulting immobilized cell particles were subjected to boiling drying at 60°C to obtain immobilized cells.

Tagatose was produced using the method of Example 2. Experimental results showed that when the immobilized *Escherichia coli* was used to catalyze consecutive rounds of reactions, the initial product yield reached up to 74%, and as the catalysis was carried out for 65 consecutive batches, the product yield was maintained at 55%.

### Example 11: Production of tagatose with immobilized Escherichia coli cells

The fermentation broth of *Escherichia coli* expressing thermostable α-glucan phosphorylase, the fermentation broth of *Escherichia coli* expressing thermostable phosphoglucomutase, the fermentation broth of *Escherichia coli* expressing thermostable phosphoglucose isomerase, the fermentation broth of *Escherichia coli* expressing thermostable tagatose 6-phosphate epimerase, and the fermentation broth of *Escherichia coli* expressing thermostable tagatose 6-phosphate phosphatase prepared in Example 9 were mixed in a ratio of 1 : 1 : 1 : 1 : 1 according to OD600, so that the OD600 = 100. And 5% w/v of diatomite was added to the bacteria suspension, and stirred homogeneously. Subsequently, 0.1% w/v of the aqueous solution of polyethyleneimine having a molecular weight of 70,000 was added and flocculation was carried out at room temperature. Then, 1% v/v of glutaraldehyde aqueous solution was added and cross-linking was carried out at room temperature for 2 hours. Filtration was carried out under vacuum to obtain a filter cake. The filter cake was extruded and thus granulated into strips having a particle size of 3.0 mm with a rotary granulator. Then the strips were broken into particles having a uniform length with a spherical shot blasting machine. The resulting immobilized cell particles were subjected to boiling drying at 75°C to obtain immobilized cells.

Tagatose was produced using the method of Example 2. Experimental results as shown in Figure 2 demonstrated that when the immobilized *Escherichia coli* was used to catalyze consecutive rounds of reactions, the initial product yield reached up to 74%, and as the catalysis was carried out for 65 consecutive batches, the product yield was maintained at 60%.

### Example 12: Production of tagatose with immobilized Escherichia coli cells

The fermentation broth of *Escherichia coli* expressing thermostable α-glucan phosphorylase, the fermentation broth of *Escherichia coli* expressing thermostable phosphoglucomutase, the fermentation broth of *Escherichia coli* expressing thermostable phosphoglucose isomerase, the fermentation broth of *Escherichia coli* expressing thermostable tagatose 6-phosphate epimerase, and the fermentation broth of *Escherichia coli* expressing thermostable tagatose 6-phosphate phosphatase prepared in Example 9 were mixed in a ratio of 1 : 1 : 1 : 1 : 1 according to OD600, so that the OD600 = 100. And 10% w/v of diatomite was added to the bacteria suspension, and stirred homogeneously. Subsequently, 1% w/v of poly(diallyldimethylammonium chloride) (PDADMAC) aqueous solution was added and flocculation was carried out at room temperature. Then, 0.5% v/v of glutaraldehyde aqueous solution was added and cross-linking was carried out at room temperature for 3 hours. Filtration was carried out under vacuum to obtain a filter cake. The filter cake was extruded and thus granulated into strips having a particle size of 1.0 mm with a rotary granulator. Then the strips were broken into particles having a uniform length with a spherical shot blasting machine. The resulting immobilized cell particles were subjected to boiling drying at 90°C to obtain immobilized cells.

Tagatose was produced using the method of Example 2. Experimental results showed that when the immobilized *Escherichia coli* was used to catalyze consecutive rounds of reactions, the initial product yield reached up to 74%, and as the catalysis was carried out for 65 consecutive batches, the product yield was maintained at 57%.

### Example 13: Production of tagatose with immobilized Escherichia coli cells

The fermentation broth of *Escherichia coli* expressing thermostable α-glucan phosphorylase, the fermentation broth of *Escherichia coli* expressing thermostable phosphoglucomutase, the fermentation broth of *Escherichia coli* expressing thermostable phosphoglucose isomerase, the fermentation broth of *Escherichia coli* expressing thermostable tagatose 6-phosphate epimerase, and the fermentation broth of *Escherichia coli* expressing thermostable tagatose 6-phosphate phosphatase prepared in Example 9 were mixed in a ratio of 1 : 1 : 1 : 5 : 5 according to OD600, so that the OD600 = 100. And 4% w/v of diatomite was added to the bacteria suspension, and stirred homogeneously. Subsequently, 1% w/v of polyethyleneimine having a molecular weight of 600 was added and flocculation was carried out at room temperature. Then, 0.3% v/v of glutaraldehyde aqueous solution was added and cross-linking was carried out at room temperature for 3 hours. Filtration was carried out under vacuum to obtain a filter cake. The filter cake was extruded and thus granulated into strips having a particle size of 0.4 mm with a rotary granulator. Then the strips were broken into particles having a uniform length with a spherical shot blasting machine. The resulting immobilized cell particles were subjected to boiling drying at 80°C to obtain immobilized cells.

Tagatose was produced using the method of Example 2. Experimental results showed that when the immobilized *Escherichia coli* was used to catalyze consecutive rounds of reactions, the initial product yield reached up to 74%, and as the catalysis was carried out for 65 consecutive batches, the product yield was maintained at 58%.

### Example 14: Production of tagatose with immobilized Escherichia coli cells

The fermentation broth of *Escherichia coli* expressing thermostable α-glucan phosphorylase, the fermentation broth of *Escherichia coli* expressing thermostable phosphoglucomutase, the fermentation broth of *Escherichia coli* expressing thermostable phosphoglucose isomerase, the fermentation broth of *Escherichia coli* expressing thermostable tagatose 6-phosphate epimerase, and the fermentation broth of *Escherichia coli* expressing thermostable tagatose 6-phosphate phosphatase prepared in Example 9 were mixed in a ratio of 1 : 1 : 1 : 1 : 1 according to OD600, so that the OD600 was at about 100. And 5% w/v of diatomite was added to the bacteria suspension, and stirred homogeneously. Subsequently, 0.8% w/v of polyethyleneimine having a molecular weight of 10,000 was added and flocculation was carried out at room temperature. Then, 0.7% v/v of tris(hydroxymethyl)phosphine aqueous solution was added and cross-linking was carried out at room temperature for 2 hours. Filtration was carried out under vacuum to obtain a filter cake. The filter cake was extruded and thus granulated into strips having a particle size of 1.0 mm with a rotary granulator. Then the strips were broken into particles having a uniform length with a spherical shot blasting machine. The resulting immobilized cell particles were subjected to boiling drying at 80°C to obtain immobilized cells.

Tagatose was produced using the method of Example 2. Experimental results showed that when the immobilized *Escherichia coli* was used to catalyze consecutive rounds of reactions, the initial product yield reached up to 75%, and as the catalysis was carried out for 65 consecutive batches, the product yield was maintained at 59%.

### Example 15: Production of tagatose with immobilized Escherichia coli cells

The fermentation broth of *Escherichia coli* expressing thermostable α-glucan phosphorylase, the fermentation broth of *Escherichia coli* expressing thermostable phosphoglucomutase, the fermentation broth of *Escherichia coli* expressing thermostable phosphoglucose isomerase, the fermentation broth of *Escherichia coli* expressing thermostable tagatose 6-phosphate epimerase, and the fermentation broth of *Escherichia coli* expressing thermostable tagatose 6-phosphate phosphatase prepared in Example 9 were mixed in a ratio of 1 : 1 : 1 : 1 : 1 according to OD600, so that the OD600 was at about 100. And 5% w/v of diatomite was added to the bacteria suspension, and stirred homogeneously. Subsequently, 0.3% w/v of polyacrylamide was added and flocculation was carried out at room temperature. Then, 1.0% v/v of N,N-methylenebisacrylamide aqueous solution was added and cross-linking was carried out at room temperature for 3 hours. Filtration was carried out under vacuum to obtain a filter cake. The filter cake was extruded and thus granulated into strips having a particle size of 1.0 mm with a rotary granulator. Then the strips were broken into particles having a uniform length with a spherical shot blasting machine. The resulting immobilized cell particles were subjected to boiling drying at 90°C to obtain immobilized cells.

Tagatose was produced using the method of Example 2. Experimental results showed that when the immobilized *Escherichia coli* was used to catalyze consecutive rounds of reactions, the initial product yield reached up to 75%, and as the catalysis was carried out for 65 consecutive batches, the product yield was maintained at 57%.

### Example 16: Production of tagatose with immobilized Escherichia coli cells

The fermentation broth of *Escherichia coli* expressing thermostable α-glucan phosphorylase, the fermentation broth of *Escherichia coli* expressing thermostable phosphoglucomutase, the fermentation broth of *Escherichia coli* expressing thermostable phosphoglucose isomerase, the fermentation broth of *Escherichia coli* expressing thermostable tagatose 6-phosphate epimerase, and the fermentation broth of *Escherichia coli* expressing thermostable tagatose 6-phosphate phosphatase prepared in Example 9 were mixed in a ratio of 1 : 1 : 1 : 1 : 1 according to OD600, so that the OD600 = 100. And 2% w/v of diatomite was added to the bacteria suspension, and stirred homogeneously. Subsequently, 0.2% w/v of the aqueous solution of polyethyleneimine having a molecular weight of 70,000 was added and flocculation was carried out at room temperature. Then, 1.0% v/v of epichlorohydrin aqueous solution was added and cross-linking was carried out at room temperature for 3 hours. Filtration was carried out under vacuum to obtain a filter cake. The filter cake was extruded and thus granulated into strips having a particle size of 1.0 mm with a rotary granulator. Then the strips were broken into particles having a uniform length with a spherical shot blasting machine. The resulting immobilized cell particles were subjected to boiling drying at 75°C to obtain immobilized cells.

Tagatose was produced using the method of Example 2. Experimental results showed that when the immobilized *Escherichia coli* was used to catalyze consecutive rounds of reactions, the initial product yield reached up to 75%, and as the catalysis was carried out for 65 consecutive batches, the product yield was maintained at 55%.

### Comparative Example 1: Production of tagatose with Bacillus subtilis cells

The fermentation broths prepared in Example 1 were centrifuged at 5,500 rpm for 10 min. The supernatants were discarded to obtain whole cells expressing thermostable α-glucan phosphorylase, whole cells expressing thermostable phosphoglucomutase, whole cells expressing thermostable phosphoglucose isomerase, whole cells expressing thermostable tagatose 6-phosphate epimerase, and whole cells expressing thermostable tagatose 6-phosphate phosphatase respectively. A 50 mM sodium phosphate buffer (pH 7.5) was added to the above cells respectively to resuspend the bacteria to OD600 = 200. The resuspended bacteria were heated at 75°C for 90 min. The above whole cells were mixed in a sodium phosphate buffer (pH 7.0) in a ratio of 1 : 1 : 1 : 1 : 1, so that OD600 = 200.

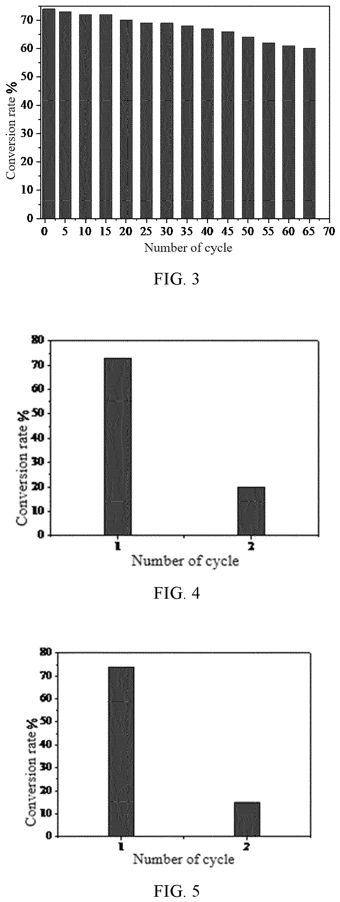

In a reaction system having a volume of 1 L, starch at a final concentration of 100 g/L, a 50 mM sodium phosphate buffer (pH 7.0) and the above mixed *Bacillus subtilis* was respectively added to achieve OD600 = 20. Reactions were allowed to carried out in a water bath in a shaker at 70°C. During reaction, the content of tagatose was analyzed by HPLC. After the reactions were completed, the bacteria were precipitated by centrifugation, washed with a buffer and then used in reactions for the next batch. Experimental results as shown in Figure 3 demonstrated that when the immobilized *Bacillus subtilis* was used to catalyze consecutive rounds of reactions, the initial product yield reached up to 73%, but as the catalysis was carried out for 2 consecutive batches, the product yield was lowered to only 20%.

### Comparative Example 2: Production of tagatose with Escherichia coli cells

The fermentation broths prepared in Example 9 were centrifuged at 5,500 rpm for 10 min. The supernatants were discarded to obtain whole cells expressing thermostable α-glucan phosphorylase, whole cells expressing thermostable phosphoglucomutase, whole cells expressing thermostable phosphoglucose isomerase, whole cells expressing thermostable tagatose 6-phosphate epimerase, and whole cells expressing thermostable tagatose 6-phosphate phosphatase respectively. A 50 mM sodium phosphate buffer (pH 7.5) was added to the above cells to resuspend the bacteria to OD600 = 200. The resuspended bacteria were heated at 75°C for 90 min. The above whole cells were mixed in a sodium phosphate buffer (pH 7.0) in a ratio of 1 : 1 : 1 : 1 : 1, so that OD600 = 200.

In a reaction system having a volume of 1 L, starch at a final concentration of 100 g/L, a 50 mM sodium phosphate buffer (pH 7.0) and the above mixed *Escherichia coli* was respectively added to achieve OD600 = 20. Reactions were allowed to carried out in a water bath in a shaker at 70°C. During reaction, the content of tagatose was analyzed by HPLC. After the reactions were completed, the bacteria were precipitated by centrifugation, washed with a buffer and then used in reactions for the next batch. Experimental results as shown in Figure 4 demonstrated that when the *Escherichia coli* was used to catalyze consecutive rounds of reactions, the initial product yield reached up to 73%, but as the catalysis was carried out for 2 consecutive batches, the product yield was lowered to only 15%.

### Comparative Example 3: Production of tagatose with immobilized permeable Bacillus subtilis cells

Recombinant *Bacillus subtilis* strains expressing a gene of thermostable α-glucan phosphorylase, a gene of thermostable phosphoglucomutase, a gene of thermostable phosphoglucose isomerase, a gene of thermostable tagatose 6-phosphate epimerase, or a gene of thermostable tagatose 6-phosphate phosphatase were selected respectively, inoculated into LB medium respectively, and cultured overnight at 37°C with shaking. Then the culture was transferred to LB medium at an inoculum volume of 1%, and cultured overnight at 37°C with shaking, centrifuged at 5,500 rpm for 10 min then with the supernatants discarded to obtain whole cells expressing thermostable α-glucan phosphorylase, whole cells expressing thermostable phosphoglucomutase, whole cells expressing thermostable phosphoglucose isomerase, whole cells expressing thermostable tagatose 6-phosphate epimerase, and whole cells expressing thermostable tagatose 6-phosphate phosphatase, respectively. A 50 mM sodium phosphate buffer (pH 7.5) was added to the above cells respectively to resuspend the bacteria to OD600 = 200. The resuspended bacteria were heated at 75°C for 90 min.

The above permeable whole cells were mixed in a sodium phosphate buffer (pH 7.0) in a ratio of 1 : 1 : 1 : 1 : 1, so that OD600 = 100. And 5% w/v of diatomite was added to the bacteria suspension, and stirred homogeneously. Subsequently, 0.5% w/v of the aqueous solution of polyethyleneimine having a molecular weight of 70,000 was added and flocculation was carried out at room temperature. Then, 0.5% v/v of glutaraldehyde aqueous solution was added and cross-linking was carried out at room temperature for 2 hours. Filtration was carried out under vacuum to obtain a filter cake. The filter cake was extruded and thus granulated into particles having a particle size of 0.4 mm with a rotary granulator. The resulting immobilized cell particles were dried at 30°C to obtain immobilized cells.

Tagatose was produced using the method of Example 2. Experimental results showed that when the immobilized permeable *Bacillus subtilis* cells were used to catalyze consecutive rounds of reactions, the initial product yield reached up to 73%, and as the catalysis was carried out for 65 consecutive batches, the product yield was 43%.

### Comparative Example 4: Production of tagatose with immobilized permeable Escherichia coli cells

Recombinant *Escherichia coli* strains expressing a gene of thermostable α-glucan phosphorylase, a gene of thermostable phosphoglucomutase, a gene of thermostable phosphoglucose isomerase, a gene of thermostable tagatose 6-phosphate epimerase, or a gene of thermostable tagatose 6-phosphate phosphatase were selected respectively, inoculated into LB medium respectively, and cultured overnight at 37°C with shaking. Then the culture was transferred to LB medium at an inoculum volume of 1%, added with IPTG for induction at 18°C and cultured overnight with shaking, centrifuged at 5,500 rpm for 10 min then with the supernatants discarded to obtain whole cells expressing thermostable α-glucan phosphorylase, whole cells expressing thermostable phosphoglucomutase, whole cells expressing thermostable phosphoglucose isomerase, whole cells expressing thermostable tagatose 6-phosphate epimerase, and whole cells expressing thermostable tagatose 6-phosphate phosphatase, respectively. A 50 mM sodium phosphate buffer (pH 7.5) was added to the above cells respectively to resuspend the bacteria to OD600 = 200. The resuspended bacteria were heated at 75°C for 90 min.

The above permeable whole cells were mixed in a sodium phosphate buffer (pH 7.0) in a ratio of 1 : 1 : 1 : 1 : 1, so that OD600 = 100. And 1% w/v of diatomite was added to the bacteria suspension, and stirred homogeneously. Subsequently, 0.5% w/v of the aqueous solution of polyethyleneimine having a molecular weight of 70,000 was added and flocculation was carried out at room temperature. Then, 1% v/v of glutaraldehyde aqueous solution was added and cross-linking was carried out at room temperature for 2 hours. Filtration was carried out under vacuum to obtain a filter cake. The filter cake was extruded and thus granulated into particles having a particle size of 1.0 mm with a rotary granulator. The resulting immobilized cell particles were dried at 30°C to obtain immobilized cells.

Tagatose was produced using the method of Example 2. Experimental results showed that when the immobilized permeable *Escherichia coli* cells were used to catalyze consecutive rounds of reactions, the initial product yield reached up to 72%, and as the catalysis was carried out for 65 consecutive batches, the product yield was 40%.

## Claims

1. A method for preparing an immobilized cell for tagatose production, **characterized in that** the method comprises the steps of:
fermenting to obtain fermentation broths of *Escherichia coli* or *Bacillus subtilis* expressing α-glucan phosphorylase, phosphoglucomutase, phosphoglucose isomerase, tagatose 6-phosphate epimerase, or tagatose 6-phosphate phosphatase respectively, and mixing the above fermentation broths to obtain a fermentation mixture;
adding an inorganic soil to the fermentation mixture, and stirring homogeneously;
further adding a flocculant to the fermentation mixture to flocculate bacteria, and then adding a cross-linking agent for cross-linking;
filtering under vacuum to obtain a filter cake, extruding the filter cake to granulate into a strip with a rotary granulator, and then breaking into particles having a uniform length with a spherical shot blasting machine; and
subjecting the resulting particles to boiling drying to obtain the immobilized cell for tagatose production.

2. A method for preparing immobilized cells for tagatose production, **characterized in that** the method comprises the steps of:
fermenting to obtain fermentation broths of *Escherichia coli* or *Bacillus subtilis* expressing α-glucan phosphorylase, phosphoglucomutase, phosphoglucose isomerase, tagatose 6-phosphate epimerase, or tagatose 6-phosphate phosphatase respectively, and mixing the above fermentation broths to obtain a fermentation mixture;
adding 1-10% w/v of inorganic soil to the fermentation mixture, and stirring homogeneously;
further adding 0.1-2% w/v of flocculant to the fermentation mixture to flocculate bacteria, then adding 0.05-3% v/v of cross-linking agent, and cross-linking for 1-4 hours;
filtering under vacuum to obtain a filter cake, extruding the filter cake to granulate into a strip with a rotary granulator, and then breaking the strip of immobilized cells into particles having a uniform length with a spherical shot blasting machine; and
subjecting the resulting particles to boiling drying to obtain the immobilized cells for tagatose production, wherein the temperature at an air inlet for the boiling drying is controlled at 60-90°C.

3. The method according to claim 1 or 2, **characterized in that** the α-glucan phosphorylase, the phosphoglucomutase, the phosphoglucose isomerase, the tagatose 6-phosphate epimerase, or the tagatose 6-phosphate phosphatase is thermostable α-glucan phosphorylase, thermostable phosphoglucomutase, thermostable phosphoglucose isomerase, thermostable tagatose 6-phosphate epimerase, or thermostable tagatose 6-phosphate phosphatase respectively.

4. The method according to claim 3, **characterized in that** the thermostable refers to having an enzymatic activity at 40°C or above.

5. The method according to claim 4, **characterized in that** the wet bacteria expressing the thermostable α-glucan phosphorylase, the thermostable phosphoglucomutase, the thermostable phosphoglucose isomerase, the thermostable tagatose 6-phosphate epimerase, and the thermostable tagatose 6-phosphate phosphatase respectively are mixed in a ratio of (0.1-10) : (0.1-10) : (0.1-10) : (0.1-10) : (0.1-10), and the bacteria suspension obtained by mixing has an OD600 of 10-150.

6. The method according to claim 1 or 2, **characterized in that** the inorganic soil is selected from montmorillonite, diatomite, kaolin or bentonite.

7. The method according to claim 1 or 2, **characterized in that** the flocculant is selected from polyethyleneimine, chitosan, poly(diallyldimethylammonium chloride), or polyacrylamide.

8. The method according to claim 7, **characterized in that** the flocculant is polyethyleneimine having a molecular weight of 600-70,000, or PDADMAC.

9. The method according to claim 1 or 2, **characterized in that** the cross-linking agent is selected from glutaraldehyde, tris(hydroxymethyl)phosphine, N,N-methylenebisacrylamide, or epichlorohydrin.

10. The method according to claim 1 or 2, **characterized in that** the method further comprises a step of sieving the obtained immobilized cells to obtain morphologically uniform immobilized cells.

11. A method for producing tagatose with an immobilized cell, **characterized in that** the method comprises converting starch or a starch derivative into tagatose with the immobilized cell obtained by the method according to any of claims 1-10.

12. The method according to claim 11, **characterized in that** the method further comprises a step of filtering and recovering the immobilized cell after reaction is completed.

13. The method according to claim 11, **characterized in that** a reaction system for biological conversion comprises 50-300 g/L of starch or starch derivative, a buffer at pH 5.0-8.0, 10-50 mM inorganic phosphate, 3-7 mM divalent magnesium ions, and the immobilized cell.

14. The method according to claim 11, **characterized in that** the buffer is an HEPES buffer, a phosphate buffer, a Tris buffer, or an acetate buffer; the inorganic phosphate is sodium phosphate or potassium phosphate.
